# EUROPÄISCHE PATENTANMELDUNG

(11) **EP 4 697 349 A1**
(43) Veröffentlichungstag der Anmeldung: **18.02.2026**
(21) Anmeldenummer: 25195835.1
(22) Anmeldetag: 14.08.2025
(51) Int. Cl.: G16H 40/20, G16H 40/63

(54) **AUTOMATISCHE OP-KONFIGURATION MITTELS ANOMALIEDETEKTION**

(30) Priorität: 16.08.2024 DE 102024123453
(71) Anmelder: Karl Storz SE & Co. KG, 78532 Tuttlingen (DE)
(72) Erfinder: KLINGENBERG, Andreas, 78532 Tuttlingen (DE)

(57) **Zusammenfassung**

Verfahren zur dynamischen Konfiguration eines integrierten Operationssaals umfassend das kontinuierliche Erfassen von Daten betreffend Geräteeinstellung, Geräteanzeige, Geräteleistung, Nutzern, Nutzeraktionen und/oder Informationen betreffend einen ablaufenden medizinischen Eingriff, das kontinuierliches Bestimmen von Eingriffssituationen basierend auf den erfassten Daten das Vergleichen aktueller Geräteeinstellungen mit Geräteeinstellungen bei entsprechenden Eingriffssituationen vergleichbarer medizinischer Eingriffe, das Ermitteln von Abweichungen der aktuellen Geräteeinstellungen von Geräteeinstellungen bei entsprechenden Eingriffssituationen vergleichbarer medizinischer Eingriffe und das Ermitteln nötiger Anpassungen der aktuellen Geräteeinstellungen, um die Abweichungen zu kompensieren.

## Beschreibung

### Technisches Gebiet

Die vorliegende Erfindung betrifft Verfahren und Vorrichtungen zum automatischen Konfigurieren eines integrierten Operationssaals, insbesondere das automatische Konfigurieren in Abhängigkeit von konkreten Eingriffssituationen.

### Hintergrund

Moderne Operationssäle sind mit verschiedensten Geräten ausgestattet. Neben den eigentlichen medizinischen Apparaten sind dies unter anderem Computer und Monitore, die Geräte steuern und/oder das Personal mit Informationen versorgen und auch Informationen aufzeichnen, Kameras, die Teil des medizinischen Eingriffs sind oder, beispielsweise in Form von Raumkameras, das Geschehen im Operationssaal aufzeichnen, aber auch Geräte, Elemente und Sensoren, die im Bereich der Haustechnik anzusiedeln sind. Verschiedene Geräte können teilweise oder vollständig miteinander in Verbindung stehen, also untereinander vernetzt sein, und so Daten austauschen. In einer einfachen Ausgestaltung in einem Operationssaal kann dies in Form eines Gerätewagens erfolgen, der als eine Art Steuereinheit für die an ihm angeschlossenen Geräte fungieren kann. In komplexeren Ausgestaltungen kommen sogenannte integrierte Operationssäle zum Einsatz, bei denen ein Computer mit anderen Komponenten oder Geräten verbunden ist und ihre Daten und/oder Funktionen zu erfassen und zu koordiniert.

Das Personal im Operationssaal hat in einem solchen integrierten System nicht nur Zugriff auf die medizinischen Informationen des Patienten, sondern kann unter anderem Geräteinformationen, wie beispielsweise Bilddaten von Endoskopkameras, anzeigen und/oder medizinische Apparate sowie Bereiche der Haustechnik, wie die Beleuchtung, steuern - und dies idealerweise von einer zentralen Bedienungsoberfläche aus.

Während die Integration die Bedienung durch das jeweilige Personal zwar vereinfacht, sind die Einstellungen als solche durch das Personal vorzunehmen und basieren auf dessen Erfahrungsschatz und auch auf dessen Wahrnehmung. Je größer die Zahl einstellbarer Parameter, umso aufwendiger ist diese Aufgabe. Um diese zu erleichtern, können beispielsweise für bestimmte Szenarien Voreinstellungen definiert werden, sodass der Benutzer durch Auswahl geeigneter Voreinstellungen oder Konfigurationen verschiedene Parameter verschiedener Geräte automatisch einstellen kann.

Hierfür können beispielsweise sehr aufwendige, statische Konfigurationen eines solchen integrierten Operationssaals implementiert werden, wobei diese Konfigurationen zum einen manuell durch den Benutzer während des Eingriffs ausgewählt werden müssen, und zum anderen gegebenenfalls händisch an konkrete Situationen oder Veränderungen angepasst werden müssen. Abweichungen, beispielsweise aufgrund neuer Eingriffsarten und/oder anderer oder neuer verwendeter Geräte, können indes nicht abgedeckt beziehungsweise im Vorfeld in der statischen Konfiguration berücksichtigt werden.

Neben dem statischen Charakter der zur Verfügung stehenden Konfigurationen werden bei den bestehenden Möglichkeiten Aspekte, die aus der Kombination von verschiedenen Geräten resultieren, regelmäßig außer Acht gelassen. Daneben werden auch Messwerte und weitergehende Informationen, die bei medizinischen Anwendungen regelmäßig und oft auch in großem Umfang erfasst werden, für die Auswahl geeigneter Geräteanpassungen regelmäßig nicht oder zumindest nicht automatisch berücksichtigt.

### Zusammenfassung der Erfindung

Entsprechend ist es eine Aufgabe der vorliegenden Erfindung, die Einschränkungen oder Defizite der aus dem Stand der Technik bekannten Verfahren, wenigstens teilweise, zu überwinden. Erfindungsgemäß wird die zugrundeliegende Aufgabe durch ein Verfahren gemäß Anspruch 1, ein System gemäß Anspruch 9 sowie ein Computerprogramm und ein Speichermedium gemäß Anspruch 10 respektive Anspruch 11 gelöst.

In einem Aspekt ist die vorliegende Erfindung auf ein Verfahren zur dynamischen Konfiguration eines integrierten Operationssaals gerichtet, wobei das Verfahren folgende Schritte umfasst: Das kontinuierliche Erfassen von Daten betreffend Geräteeinstellung, Geräteanzeige, Geräteleistung, Nutzern, Nutzeraktionen und/oder Informationen, die einen ablaufenden medizinischen Eingriff betreffen; das kontinuierliche Bestimmen von Eingriffssituationen basierend auf den erfassten Daten; das Vergleichen aktueller Daten mit Daten bei entsprechender Eingriffssituation vergleichbarer medizinischer Eingriffe; das Ermitteln von Abweichungen aktueller Daten von Daten bei entsprechender Eingriffssituation vergleichbarer medizinischer Eingriffe; sowie das Ermitteln nötiger Anpassungen der aktuellen Geräteeinstellungen um die Abweichungen zu kompensieren. Beispiele für integrierte Geräte im Sinne der vorliegenden Erfindung sind insbesondere Medizingeräte, OP-Beleuchtung, Dokumentationssysteme, Monitore, Kameras, Belüftungssysteme, Klimatisierungssystem, Netzwerkkomponenten, insbesondere zum Empfang und Versenden von Daten, Kommunikationskomponenten, sowie verschiedene Arten von Sensoren und dergleichen. Erfindungsgemäß können die Schritte des Bestimmens der Eingriffssituation, das Vergleichen von Daten und das Ermitteln von Abweichungen auf den gesamten erfassten Datensatz oder auf Teile des Datensatzes beruhen, wobei die einzelnen Schritte insbesondere auch unterschiedliche, sich gegebenenfalls überlappende Bereiche der Daten betreffen können. In bevorzugten Ausführungsformen können einzelne Schritte auch sequenziell oder gleichzeitig auf unterschiedliche, sich gegebenenfalls überlappende Bereiche der Daten angewandt werden. So kann beispielsweise in einem ersten Schritt durch den Vergleich der Daten eine Abweichung von einem normalen Ablauf eines medizinischen Eingriffs, eine sogenannte Anomalie, erkannt werden. In einem nachfolgenden Schritt können, insbesondere wenn eine Anomalie erkannt wurde, einzelne Datenbereiche, wie beispielsweise Geräteeinstellungen, verglichen werden, um so gegebenenfalls mögliche Gründe für die Anomalie in Form von abweichenden Geräteeinstellungen zu identifizieren. Grundsätzlich können im Sinne der vorliegenden Erfindung jedoch auch nur Geräteeinstellungen verglichen und Unregelmäßigkeiten also allein aufgrund abweichender Geräteeinstellungen ermittelt werden.

In einer Ausführungsform des erfindungsgemäßen Verfahrens kann die kontinuierliche Bestimmung der Eingriffssituation auf Nutzeraktionen, insbesondere auf entsprechende Benutzereingaben basieren. Hierbei können die Benutzereingaben insbesondere über eine entsprechende Benutzeroberfläche erfasst oder auch über Spracherkennung aus verbalen Instruktionen seitens der Benutzer erfasst werden. Alternativ oder in Ergänzung dazu können auch über geeignete Bilderkennungsalgorithmen bestimmte Handlungen und/oder Bewegungsabläufe erkannt und daraus die jeweilige Eingriffssituation abgeleitet werden.

In einer bevorzugten Ausführungsform des erfindungsgemäßen Verfahrens kann die Bestimmung der Eingriffssituation den Vergleich aktueller Daten mit historischen Daten umfassen. Vorzugsweise kann die Bestimmung der aktuellen Eingriffssituation unter Verwendung eines unter Verwendung historischer Daten trainierten, auf künstlicher Intelligenz basierenden Erkennungsalgorithmus erfolgen. Hierbei können Datensätze, insbesondere annotierte Datensätze historischer medizinischer Eingriffe verwendet werden, wobei vorzugsweise Daten eines vergleichbaren Operationssaals bzw. Setups verwendet werden. Insbesondere kann bei der Ermittlung der Eingriffssituation darauf abgestellt werden, dass wenigstens teilweise Daten gleicher oder vergleichbarer Geräte zugrunde gelegt werden. Vorzugsweise kann die Ermittlung auch Verwendung von verfügbaren den Eingriff betreffenden Informationen, wie beispielsweise Informationen über die Art des medizinischen Eingriffs aus einem OP-Plan oder aus der Akte des Patienten umfassen. Gleichermaßen kann bei der Ermittlung die Art des Operationssaals oder Zuordnung des Operationssaals zu einer bestimmten Abteilung berücksichtigt werden. Entsprechend kann etwa die Zuordnung eines Operationssaals zu einer orthopädischen Abteilung eines Krankenhauses oder zu einer dermatologischen Abteilung eines Krankenhauses die Art der möglichen medizinischen Eingriffe signifikant einschränken und so zu einer verbesserten Ermittlung der Eingriffssituation frühen, da bestimmte Eingriffssituationen nur bei bestimmten medizinischen Eingriffen zu erwarten sind.

In einer weiteren Ausführungsform des erfindungsgemäßen Verfahrens kann die Eingriffssituation auch auf den ausführenden Operateur und/oder auf das Operationsteam abstellen. Hierbei kann der Operateur und/oder das Team erfindungsgemäß aus den entsprechenden Informationen aus einem Operationssaal-Belegungsplan, aus der Krankenakte des Patienten oder vergleichbaren Informationen bestimmt werden. Alternativ oder in Ergänzung dazu kann der Operateur über eine Personenerkennung, insbesondere über eine Gesichtserkennung bestimmt werden. Hierbei können insbesondere Bilddaten einer Kamera verwendeten werden, die in einem dem Operationssaal zugeordneten Bereich, in dem das Gesicht gut erkennbar ist, also beispielsweise noch kein Mundschutz getragen wird, erfasst werden. Alternativ oder in Ergänzung dazu kann der Operateur und/oder das Operationsteam auch über eine entsprechende Benutzereingabe über eine Benutzeroberfläche erkannt werden. Entsprechend können beispielsweise persönliche Vorlieben oder Bedürfnisse bei der Konfiguration berücksichtigt werden oder auch Informationen über den Eingriff abgeleitet werden.

In einer weiteren Ausführungsform des erfindungsgemäßen Verfahrens können die ermittelten nötigen Anpassungen wenigstens teilweise automatisch vorgenommen werden. Entsprechend können, je nach Integrationsgrad, aber auch in Abhängigkeit von möglichen direkten Auswirkungen auf den medizinischen Eingriff oder von rechtlichen Erfordernissen, bestimmte Änderungen automatisch vorgenommen werden, während andere Änderungen wenigstens die Freigabe durch das Personal erfordern. Entsprechend können bestimmte Anpassungen, wie beispielsweise Anpassungen der Beleuchtung, der Belüftung, der Klimatisierung und dergleichen, vorzugsweise innerhalb bestimmter, vordefinierter Grenzen, automatisch erfolgen. Änderungen an den Einstellungen medizinische Geräte, die nicht automatisch durchgeführt werden dürfen oder können, können nicht automatisch und im Hintergrunderfolgen.

Entsprechend kann in einer weiteren Ausführungsform des erfindungsgemäßen Verfahrens kann die automatische Vornahme der Anpassungen nach Freigabe erfolgen. Entsprechend können Anpassungen an bestimmten Geräten, insbesondere an medizinischen Geräten oder Anpassungen, die über bestimmte, vordefinierte Grenzen hinausgehen, über ein entsprechendes Ausgabemedium beziehungsweise Ausgabegerät, wie beispielsweise einen Monitor oder ein Gerätedisplay angezeigt beziehungsweise vorgeschlagen werden. Die eigentliche Anpassung kann dann entweder manuell durch das Personal oder automatisch nach entsprechender Freigabe bzw. Bestätigung durch das Personal erfolgen. Die Freigabe bzw. Bestätigung kann hierbei über eine entsprechende Benutzereingabe an einer Benutzeroberfläche oder auch über Spracherkennung entsprechender verbaler Instruktionen erfolgen.

In einer weiteren Ausführungsform des erfindungsgemäßen Verfahrens kann die Ermittlung von Abweichungen durch Vergleich von erfassten Daten mit Daten bei entsprechender Eingriffssituation vergleichbarer medizinischer Eingriffe basierend auf historischen Daten erfolgen. Insbesondere kann hierbei unter Verwendung verschiedener statistischer Methoden die Signifikanz ermittelter Abweichungen von Geräteeinstellungen bestimmt werden und entsprechenden Anpassungen nur bei entsprechender Signifikanz der Abweichung durchgeführt oder vorgeschlagen werden. Hierbei können entsprechende Signifikanzwerte geräte- und/oder einstellungsabhängig vorbestimmt und an entsprechender Stelle hinterlegt werden. Alternativ oder in Ergänzung dazu können auch künstliche Daten entsprechender Eingriffssituationen vergleichbarer medizinischer Eingriffe verwendet werden. Insbesondere können diese künstlichen Daten Geräteeinstellungen gemäß den Vorgaben der Gerätehersteller oder empirisch oder theoretisch bestimmte, bevorzugte Geräteeinstellungen für bestimmte Anwendungen umfassen.

In einer weiteren Ausführungsform des erfindungsgemäßen Verfahrens kann die Ermittlung von Abweichungen wenigstens teilweise auf einem auf künstliche Intelligenz gestützten Erkennungsalgorithmus erfolgen. In Ergänzung dazu kann der Erkennungsalgorithmus mit historischen Daten trainiert werden oder worden sein. Auch hierbei kann unter Verwendung verschiedener statistischer Methoden die Signifikanz ermittelter Abweichungen von Geräteeinstellungen bestimmt werden und entsprechende Anpassungen bei entsprechender Signifikanz der Abweichung durchgeführt oder vorgeschlagen werden. Hierbei können entsprechende Signifikanzwerte geräte- und/oder einstellungsabhängig vorbestimmt und an entsprechender Stelle hinterlegt werden. In bevorzugten Ausführungsformen kann der auf künstliche Intelligenz gestützte Erkennungsalgorithmus unter Berücksichtigung aller verfügbaren Daten, insbesondere auch unter Berücksichtigung von Sensordaten, Szenarien erkennen. Insbesondere kann basierend auf Sensordaten, beispielsweise aufgrund akustischer Signale, wie beispielsweise die Kommunikation im Operationssaal, und der Art der Bewegungen im Operationssaal, ein Stresslevel erkannt werden, auf das gegebenenfalls mit Anpassungen reagiert werden kann. Entsprechend kann etwa die Beleuchtung und/oder die Temperatur angepasst werden.

In einem weiteren Aspekt ist die vorliegende Erfindung auf ein System, umfassend wenigstens eine Datenquelle in einem integrierten Operationssaal, wenigstens ein konfigurierbares Gerät in dem integrierten Operationssaal, und wenigstens eine Datenverarbeitungseinheit gerichtet, wobei das System konfiguriert ist, wenigstens eines der vorstehend beschriebenen Verfahren auszuführen. Datenquellen im Sinne der vorleigenden Erfindung können Geräte und/oder Sonsoren sein, die Daten an die Datenverarbeitungseinheit liefern könne. Ein System im Sinne der vorliegenden Erfindung kann hierbei beliebig viele konfigurierbare Geräte und Datenquellen umfassen. In bevorzugten Ausführungsformen kann ein System neben der wenigstens eine Datenverarbeitungseinheit und dem wenigstens einen konfigurierbaren Gerät auch wenigstens einen Sensor zur Erfassung verschiedener Parameter, die gegebenenfalls zur Erkennung der Eingriffssituation oder zur indirekten Erkennung von Geräteeinstellungen verwendet werden können, umfassen. Entsprechende Sensoren können Helligkeitssensoren, Temperatursensoren, Feuchtigkeitssensoren, Kameras, insbesondere Raumkameras, Mikrofone und dergleichen umfassen.

In einem weiteren Aspekt ist die vorliegende Erfindung auf ein Computerprogramm gerichtet, wobei das Computerprogramm Befehle umfasst, die bei der Ausführung des Programms durch einen Computer diesen veranlassen, wenigstens eines der vorstehend beschriebenen Verfahren auszuführen.

In einem weiteren Aspekt ist die vorliegende Erfindung auf ein computerlesbares Speichermedium gerichtet, wobei das Speichermedium Befehle speichert, die bei der Ausführung durch einen Computer diesen veranlassen, wenigstens eines der vorstehend beschriebenen Verfahren auszuführen.

### Kurzbeschreibung der Abbildungen

Die beiliegenden Figuren veranschaulichen beispielhafte Ausführungsformen und Anwendungen der vorliegenden Erfindung. Hierbei zeigen:
Abbildung 1 zeigt schematisch den Ablauf des erfindungsgemäßen Verfahrens;
Abbildung 2 zeigt schematisch den Datenfluss des erfindungsgemäßen Verfahrens;
Abbildung 3 zeigt schematisch ein erfindungsgemäßes System;
Abbildung 4 zeigt einen integrierten Operationssaal, in dem das System/ Verfahren implementiert ist.

### Detaillierte Beschreibung

Die vorliegende Erfindung wird nun unter Bezugnahme auf die beiliegenden Abbildungen näher beschrieben. Die Erfindung kann jedoch in vielen verschiedenen Formen verkörpert werden und sollte nicht auf die hier dargestellten Ausführungsformen beschränkt ausgelegt werden. Es sei angemerkt, dass die Abbildungen allgemeine Merkmale der in den jeweiligen Ausführungsformen genutzten Vorrichtungen und Verfahren veranschaulichen. Diese Abbildungen geben jedoch möglicherweise nicht die exakte Struktur oder das exakte Merkmal einer gegebenen Ausführungsform wieder.

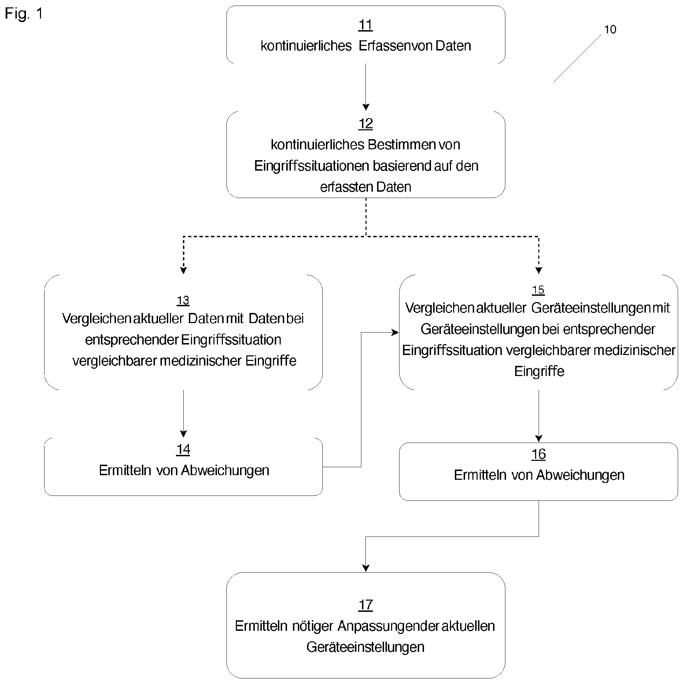

Abbildung 1 zeigt hierbei schematisch den Ablauf des erfindungsgemäßen Verfahrens 10, wobei darauf hingewiesen wird, dass die Reihenfolge der einzelnen Verfahrensschritte grundsätzlich nicht vorgegeben ist und sich im Wesentlichen durch das zumindest teilweise aufeinander Aufbauen ergibt; insbesondere laufen manche Verfahrensschritte kontinuierlich ab, sodass Ergebnisse dieser Verfahrensschritte zwar Voraussetzung für einen nachfolgenden Verfahrensschritt sein können, die jeweiligen Ergebnisse den vorhergehende Verfahrensschritt jedoch nicht zwangsläufig abschließen oder beenden.

In einem Schritt 11 erfolgt das kontinuierliche Erfassen von Daten von allen verfügbaren Datenquellen. Hierbei können Daten von reinen Datenquellen, wie Sensoren oder Datenbanken, erfasst werden, aber auch Daten von konfigurierbaren Geräten, wobei diese sowohl Geräteeinstellungsinformationen wie durch die Geräte erfasste Messwerte umfassen können.

In einem Schritt 12 erfolgt das kontinuierliche Bestimmen von Eingriffssituationen basierend auf den erfassten Daten. Die Bestimmung der Eingriffssituation erfolgt hierbei auf den erfassten Daten, wobei die erfassten Daten auch entsprechende Eingabe durch den Benutzer umfassen können. Bevorzugt erfolgt die Bestimmung der Eingriffssituation unter Verwendung von auf künstliche Intelligenz basierende Algorithmen, wobei insbesondere Informationen die Art des medizinischen Eingriffs betreffend, berücksichtigt werden können.

In einem Schritt 13 erfolgt der Vergleich aktueller Daten mit Daten bei entsprechenden, insbesondere historischen, Eingriffssituation vergleichbarer medizinischer Eingriffe und in einem nachfolgenden Schritt 14 erfolgt die Ermittlung von Abweichungen zwischen den beiden Datensätzen. Hierbei sei betont, dass die 12, 13 und 14 den gesamten erfassten Datensatz oder auf Teile des erfassten Datensatzes betreffen können, wobei die einzelnen Schritte insbesondere auch unterschiedliche, sich gegebenenfalls überlappende Bereiche der Daten betreffen können. Insbesondere können die Schritte des Vergleichens von Daten und des Ermittelns von Abweichungen gleichzeitig oder sequenziell auf unterschiedliche, sich gegebenenfalls überlappende Bereiche der Daten angewandt werden. So kann beispielsweise in einem ersten Schritt 13 der Vergleich der Daten mit Daten vergleichbarer medizinischer Eingriffe erfolgen und in einem Schritt 14 gegebenenfalls eine Abweichung von einem normalen Ablauf eines medizinischen Eingriffs, eine sogenannte Anomalie, erkannt werden. In einem nachfolgenden Schritt 15 können, insbesondere, wenn eine Anomalie erkannt worden ist, einzelne Datenbereiche, vorzugsweise die Geräteeinstellungen, mit Geräteeinstellungen vergleichbarer medizinischer Eingriffe verglichen werden, um so gegebenenfalls möglichen Gründe für die Anomalie in Form von abweichenden Geräteeinstellungen in einem Schritt 16 zu identifizieren. Alternativ können auch ohne vorherige Durchführung der Schritte 13 und 14 nur in Schritt 15 Geräteeinstellungen verglichen und in Schritt 16 dann Unregelmäßigkeiten beziehungsweise Abweichungen allein aufgrund abweichender Geräteeinstellungen ermittelt werden.

Grundsätzlich erfolgt das erfindungsgemäße Ermitteln von Abweichungen aktueller Daten, wie beispielsweise von Geräteeinstellungen, von Referenzdaten, wie beispielsweise Geräteeinstellungen bei entsprechender Eingriffssituation vergleichbarer medizinischer Eingriffe, den Prinzipien der Anomalieerkennung. Es werden also Datenpunkte in einem Datensatz identifiziert, die von der Norm, also von als normal kategorisierten oder gemittelten Daten entsprechender, historischer Eingriffsituationen, abweichen. Bei Verwendung auf künstliche Intelligenz basierender Algorithmen markiert die Anomalieerkennung Instanzen, die von den üblichen Mustern oder statistischen Modellen im Großteil abweichen. Durch die Erkennung von Abweichungen / Anomalien können potenzielle Probleme automatisch erkannt und gegebenenfalls behoben werden.

Bei der Anomalieerkennung kann zunächst ein sogenanntes Baseline-Verhaltensprofil erstellt werden; erfindungsgemäß kann hierbei insbesondere die Art des medizinischen Eingriffs berücksichtigt werden, wodurch die Erkennung von Mustern verbessert werden kann. Dieses Profil bildet die erwarteten Muster und Verhaltensweisen der Daten ab, wenn der medizinische Eingriff erwartungsgemäß abläuft und kann entsprechend auf Referenzdaten basieren. Dazu können historische Daten, eine repräsentative Probe des normalen Verhaltens oder auch Simulationsdaten verwendet werden. Erfindungsgemäß können Verhaltensprofile für verschiedenen medizinischen Eingriffe, gegebenenfalls mit höherer Granularität auch für Eingriffssituationen der jeweiligen medizinischen Eingriffe, insbesondere im Vorfeld erstellt und an geeigneterer Stelle gespeichert werden.

Die während des medizinischen Eingriffs erfassten Daten können - vollständig oder teilweise - mit einem oder mehreren entsprechenden Profilen in Referenzdaten verglichen werden. Insbesondere kann hier die Art des medizinischen Eingriffs, wie er aus den erfassten Daten bestimmt oder aus Benutzereingaben erfasst werden kann, bei der Auswahl geeigneter Vergleichsdaten berücksichtigt werden. Gleichermaßen können auch Aspekte wie die Ausstattung des Operationssaals, die vorhandenen Datenquellen, die Zusammensetzung des Operationsteams bei der Auswahl geeigneter Referenzdaten berücksichtigt werden. Die Daten werden ausgewertet, und ihre Übereinstimmung mit den erwarteten Eigenschaften des normalen Verhaltensprofils wird gemessen. Alle Datenpunkte, die signifikant davon abweichen, werden als Anomalien markiert. Diese Abweichungen können mit verschiedenen statistischen Techniken, auf künstliche Intelligenz basierende Algorithmen oder regelbasierten Ansätzen, wie sie dem Fachmann bekannt sind, identifiziert werden.

Sofern die Anomalien Abweichungen der Eingriffssituationen also solche darstellen, also Unregelmäßigkeiten im Ablauf des medizinischen Eingriffs bedeuten, können diese genauer untersucht werden, um deren Ursachen und gegebenenfalls deren Auswirkungen zu ermitteln. Insbesondere können, wie vorstehend beschrieben durch einen Vergleich von Geräteeinstellungen, Abweichungen auf dieser Ebene identifiziert werden und in einem Schritt 17 entsprechenden Anpassungen der Geräteeinstellungen ermittelt und nachfolgend vorgeschlagen oder durchgeführt werden.

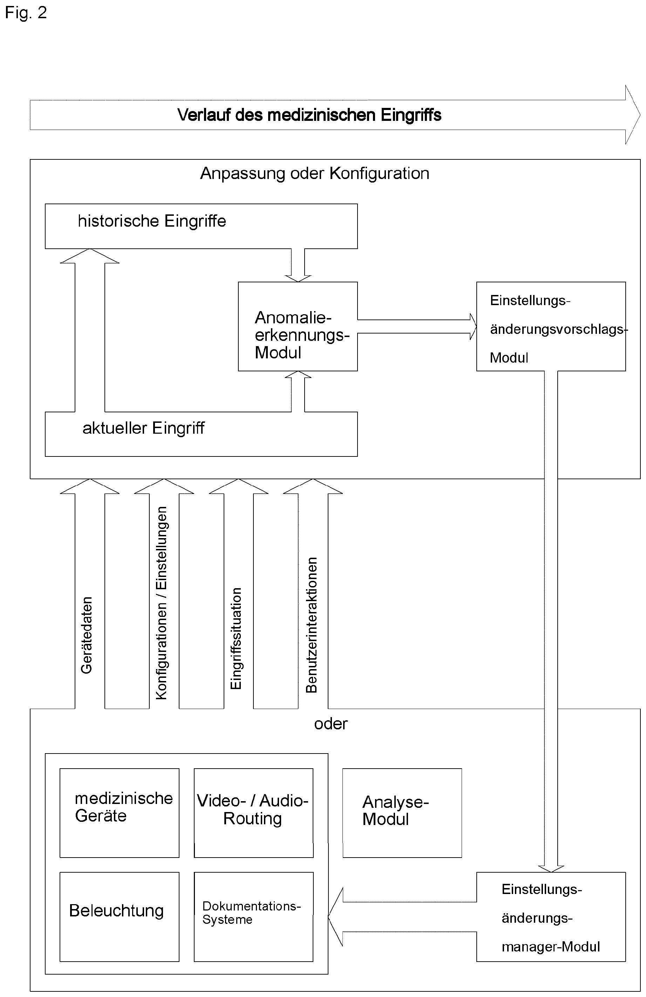

Abbildung 2 zeigt schematisch den Ablauf beziehungsweise den Datenfluss des erfindungsgemäßen Verfahrens. Die verschiedenen, die einzelnen Verfahrensschritte ausführenden Module sind erfindungsgemäß als Software-Module auf einem oder mehreren Computern implementiert. Während eines aktuellen medizinischen Eingriffs werden kontinuierlich verschiedenen Daten erfasst. Wie in Abbildung 2 gezeigt, können diese Daten verschiedene, direkt oder indirekt mit dem Eingriff in Verbindung stehende Daten umfassen. Insbesondere können diese Daten Gerätedaten, d. h. Daten von in das System integrierten medizinischen und nicht medizinischen Geräten, von Sensoren erfasste Daten, Konfigurations- und Einstellungsinformationen integrierter Geräte, die Eingriffssituation betreffende Daten, In Datenbanken hinterlegte Informationen, sowie Benutzerinteraktionen betreffende Daten umfassen. All diese den aktuellen Eingriff betreffenden Daten werden erfindungsgemäß kontinuierlich durch ein Anomalieerkennungs-Modul mit historischen Daten, basierend auf den vorstehenden Grundprinzipien der Anomalieerkennung, verglichen. Alternativ oder in Ergänzung dazu können auch Simulationsdaten und/oder Daten basierend auf Herstellervorgaben verwendet werden. Wie vorstehend beschrieben, können Abweichungen / Anomalien mit verschiedenen statistischen Techniken, auf künstliche Intelligenz basierende Algorithmen oder regelbasierten Ansätzen, wie sie dem Fachmann bekannt sind, identifiziert werden.

Wenn eine Abweichung /Anomalie erkannt wurde, können mittels eines Einstellungsänderungsvorschlags-Moduls über den Vergleich von Geräteeinstellungen des aktuellen Eingriffs mit solchen historischer Eingriffe und/oder Herstellervorgaben Vorschläge für Änderungen der Geräteeinstellungen ermittelt werden. Diese Änderungen der Geräteeinstellungen können, wie in Abbildung 2 gezeigt, beispielsweise Einstellungen medizinischer Geräte, allgemeine Operationssaaleinstellungen, wie Änderungen der Beleuchtung, Einstellungen, die das Video- und/oder Audio-Routing betreffen und auch Anweisungen an ein Dokumentationssystem umfassen. Erfindungsgemäß sind die Vorschläge für Einstellungsänderungen nicht auf die vorstehend genannten Funktionen oder Geräte beschränkt und können entsprechend jedwede integrierte Funktion und jedwedes integrierte Gerät betreffen.

Die Vorschläge für Einstellungsänderungen können wie in Abbildung 2 gezeigt an ein Einstellungsänderungsmanager-Modul weitergegeben werden. Je nach Art der vorgeschlagenen Einstellungsänderung kann diese durch das Einstellungsänderungsmanager-Modul entweder direkt an das entsprechende Gerät weitergegeben beziehungsweise direkt umgesetzt werden oder auf einem entsprechenden Ausgabegerät dargestellt, also vorgeschlagen werden. Im letzteren Fall kann die vorgeschlagene Einstellungsänderung entweder durch das Personal manuell vorgenommen werden oder vom Einstellungsänderungsmanager-Modul nach Freigabe durch das Personal zur automatischen Umsetzung an das jeweilige Gerät weitergegeben werden. Die Entscheidung, wie mit vorgeschlagenen Einstellungsänderungen verfahren wird, also ob diese direkt umgesetzt werden können oder ob sie der Freigabe durch das Personal bedürfen, kann mithilfe eines mit Analysemoduls getroffen werden.

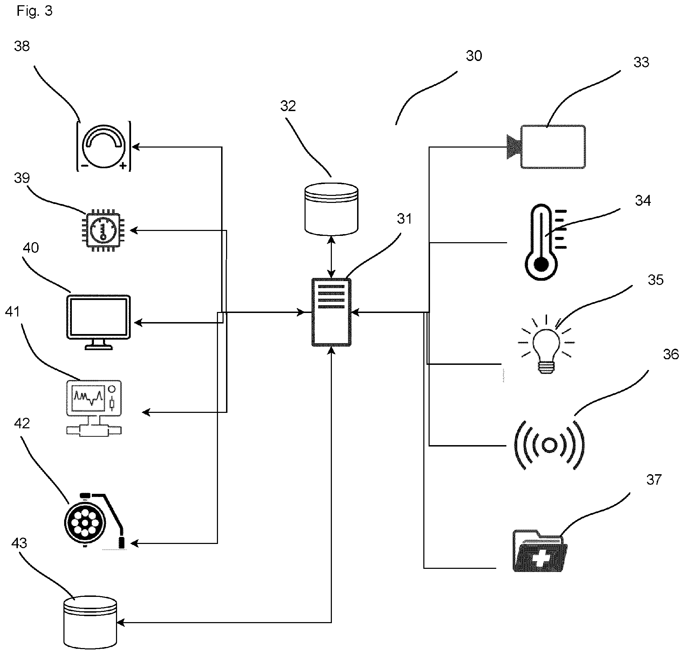

Abbildung 3 zeigt ein erfindungsgemäßes System 30 zur Durchführung des erfindungsgemäßen Verfahrens. Das System umfasst eine Datenverarbeitungseinheit 31, die mit einem Datenspeicher 32 verbunden ist. Der Datenspeicher speichert die für die Anomalieerkennung zu verwendenden Informationen, also Referenzdaten, Regelwerke, Trainingsdaten für auf künstliche Intelligenz basierende Algorithmen, die trainierten Algorithmen und dergleichen. Das System 30 umfasst verschiedene Datenquellen, wie eine Raumkamera 33, einen Temperatursensor 34, einen Helligkeitssensor 35, einen weiteren, allgemeinen Sensor 36 sowie Zugriff auf digitale Krankenhausdaten 37, wie beispielsweise Patientenakten und/oder einen Operationssaal-Belegungspläne. Das System 30 umfasst weiter verschiedene konfigurierbare Geräte, wie einen Helligkeitsregler 38, einen Temperaturregler 39, einen Monitor 40, ein medizinisches Gerät 41, eine Operationslampe 42 und ein Dokumentationssystem 43. Alle vorstehenden genannten Datenquellen und Geräte sind operativ mit der Datenverarbeitungseinheit verbunden und bilden so die Grundlage eines integrierten Operationssaals. Es sei erwähnt, dass die genannten Datenquellen und Geräte lediglich eine beispielhafte Auflistung darstellen. Es sei auch erwähnt, dass erfindungsgemäß die konfigurierbaren Geräte selbst auch als Datenquellen fungieren, sie es, dass sie Daten, beispielsweise medizinische Daten, erfassen oder sei es, dass ihre jeweiligen Geräteeinstellungen ausgelesen werden.

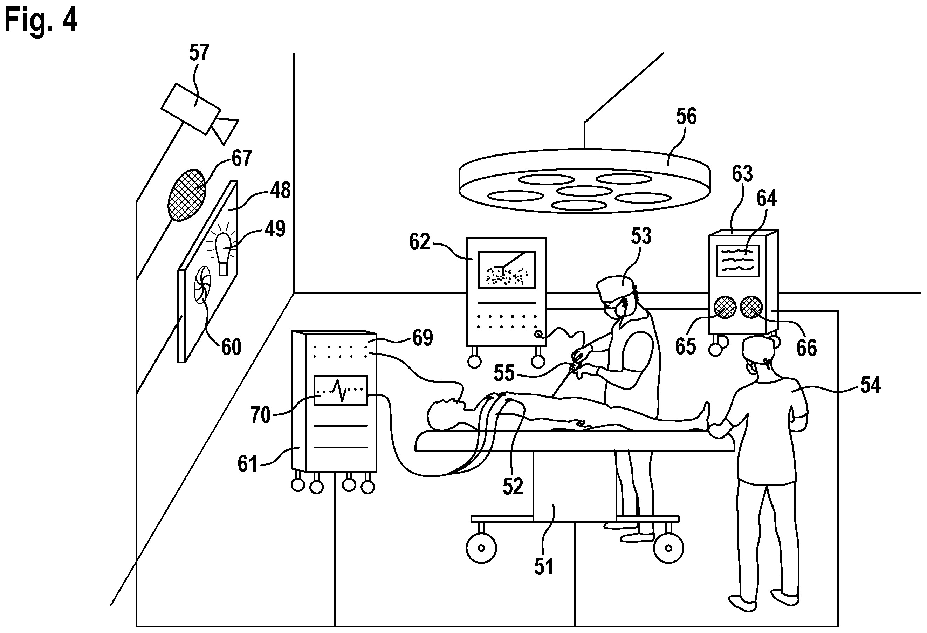

Abbildung 4 zeigt die Implementierung und Anwendung des erfindungsgemäßen Verfahrens beziehungsweise Systems in einem integrierten Operationssaal, in dem beispielhaft ein minimalinvasiver Eingriff vorgenommen wird. In dem Operationssaal befindet sich in dem dargestellten Szenario ein Operationstisch 51, auf dem ein Patient 52 liegt. Der Patient 52 ist intubiert und wird über einen Schlauch 68, der in die Luftröhre eingeführt wurde, mittels eines Beatmungsgeräts 69 künstlich beatmet. Über das Beatmungsgerät kann die Atmungsfunktion des Patienten überwacht und reguliert werden. Der Patient 52 ist ferner über Elektroden an ein EKG-Gerät angeschlossen 70. Beatmungsgerät 69 und EKG-Gerät 70 sind zur Vereinfachung als gemeinsames Kombigerät 61 dargestellt. Der Operationssaal weist ferner ein Beleuchtungssystem 56, eine Raumkamera 57, eine Steuereinheit 48 mit Elementen zur Beleuchtungssteuerung 59 und Lüftungssteuerung 60 sowie eine Lautsprecher-Mikrofon-Kombination 67 auf. Im Operationssaal befinden sich in der Darstellung ein Operateur 53 sowie eine OP-Schwester 54. Bei dem Patienten 52 wird durch den Operateur 53 ein minimalinvasiver Eingriff unter Verwendung eines Endoskops 55 im Bauchraum durchgeführt. Das Endoskop 45 ist mit einer entsprechenden Endoskopsteuereinheit 62 verbunden, mit der verschiedene Funktionen, wie beispielsweise elektrochirurgische Funktionen ausgeführt werden können und auf der eine Darstellung der mit der Endoskopkamera aufgenommenen Bilder auf einem Bildschirm erfolgen. In dem Operationssaal befindet sich ferner eine Steuereinheit 63, umfassend einen Bildschirm 64, eine Mikrofoneinheit 65 und eine Lautsprechereinheit 66, die konfiguriert ist, um das erfindungsgemäße Verfahren auszuführen. Die Steuereinheit ist mit allen anderen Geräten im Operationssaal, insbesondere mit der dem Operationstisch 51, der Raumkamera 57, der Steuereinheit 58, das Kombigerät 61, der Lautsprecher-Mikrofon-Kombination 67 und der Endoskopsteuereinheit 62 verbunden, sodass Geräteeinstellungen und zusätzliche Informationen direkt und/oder indirekt, wie vorstehend in Detail beschrieben, erfasst werden können. Diese Integration ermöglicht - neben der vereinfachten Bedienung durch das Personal - auch eine zentrale Überwachung der verschiedenen, verfügbaren Geräte und gegebenenfalls sogar eine zentrale, gegebenenfalls sogar automatisierte Konfiguration einzelner Geräte.

Auf der Steuereinheit 63 sind in dem dargestellten Beispiel die verschiedenen, das erfindungsgemäße Verfahren ausführenden Module implementiert, wobei grundsätzlich auch andere Konfigurationen von der Erfindung umfasst sind. Entsprechend werden von der Steuereinheit 63 kontinuierlich von den verbundenen, integrierten Geräten und Sensoren Daten erfasst. Die Steuereinheit 63 ist operativ mit einer Speichereinheit verbunden, auf der die für die Anomalieerkennung zu verwendenden Daten gespeichert sind. Die Speichereinheit kann hierbei in die Steuereinheit 63 integriert oder extern, gegebenenfalls Cloud-basiert, ausgestaltet sein. Die Daten können historische Daten von medizinischen Eingriffen, simulierte Daten, aber auch auf künstliche Intelligenz basierende, trainierte Algorithmen umfassen.

Sobald von der Steuereinheit eine Unregelmäßigkeit oder Anomalie erkannt wird, werden, wie vorstehend beschrieben, mögliche Änderungen von Geräteeinstellungen bestimmt und entweder direkt umgesetzt oder auf der Ausgabeeinheit 64 angezeigt und so den Personal vorgeschlagen. Beispielsweise kann, wenn erkannt wird, dass die Operationslampe 56 dunkler ist als normalerweise bei vergleichbaren Eingriffen, diese automatisch heller gestellt werden. Wenn aber aufgrund der erfassten Daten der durchführende Operateur identifiziert wurde und dieser bei seinen bisherigen Eingriffen mit einer geringeren Beleuchtung arbeitet, kann die Einstellung der Operationslampe unverändert bleiben.

Gleichermaßen kann basierend auf den vom Beatmungsgerät 69 stammenden Daten eine Unregelmäßigkeit bei der Beatmung erkannt werden. Die Steuereinheit 63 kann in diesem Fall auf der Ausgabeeinheit 64 einen Vorschlag für eine Änderung der Einstellungen des Beatmungsgeräts 69 vorschlagen und um Freigabe der Änderung durch das Personal ersuchen. Das Personal kann die entsprechende Freigabe über eine Eingabe oder auch über einen Sprachbefehl erteilen, wodurch die Steuereinheit 63 die entsprechende, vorgeschlagene Änderung automatisch vornimmt.

In gleicher Weise können sämtliche erfasste Daten kontinuierlich auf Abweichungen und/oder Anomalien geprüft werden, und gegebenenfalls Vorschläge für Änderungen von Geräteeinstellungen bestimmt werden.

Entsprechend dieser beiden Beispiel können an sämtlichen einstellbaren integrierten Geräten Änderungen an den Geräteeinstellungen direkt oder indirekt vorgenommen werden.

Der Umfang dieser Offenbarung schließt sämtliche Änderungen, Ersetzungen, Variationen, Abwandlungen und Modifikationen an den hierin beschriebenen oder veranschaulichten Ausführungsbeispielen ein, die sich dem Fachmann in naheliegender Weise daraus ergeben würden. Der Umfang dieser Offenbarung ist nicht auf die hierin beschriebenen oder veranschaulichten Ausführungsbeispiele beschränkt. Obwohl diese Offenbarung bestimmte Ausführungsformen hierin als bestimmte Komponenten, Elemente, Merkmale, Funktionen, Operationen oder Schritte umfassend beschreibt und veranschaulicht, können zudem beliebige dieser Ausführungsformen beliebige Kombinationen oder Permutationen beliebiger Komponenten, Elemente, Merkmale, Funktionen, Operationen oder Schritte umfassen, die sich dem Fachmann in naheliegender Weise daraus ergeben würden. Ein Verweis in den beigefügten Patentansprüchen darauf, dass ein Verfahren, eine Vorrichtung oder eine Komponente einer Vorrichtung oder ein System zum Durchführen einer bestimmten Funktion angepasst, eingerichtet, fähig, konfiguriert, befähigt, betriebsfähig oder betriebsbereit ist, schließt darüber hinaus dieses Gerät, dieses System oder diese Komponente ein, unabhängig davon, ob es oder sie bzw. diese bestimmte Funktion aktiviert, eingeschaltet oder freigegeben ist, solange dieses Gerät, dieses System oder diese Komponente dazu angepasst, eingerichtet, fähig, konfiguriert, befähigt, betriebsfähig oder betriebsbereit ist. Auch wenn diese Offenbarung bestimmte Ausführungsformen als vorteilhaft beschreibt oder veranschaulicht, können zudem bestimmte Ausführungsformen keine, einige oder alle diese Vorteile bereitstellen.

## Patentansprüche

1. Verfahren zur dynamischen Konfiguration eines integrierten Operationssaals umfassend:
kontinuierliches Erfassen von Daten betreffend Geräteeinstellung, Geräteanzeige, Geräteleistung, Nutzern, Nutzeraktionen und/oder Informationen betreffend einen ablaufenden medizinischen Eingriff;
kontinuierliches Bestimmen von Eingriffssituationen basierend auf den erfassten Daten;
Vergleichen aktueller Geräteeinstellungen mit Geräteeinstellungen bei entsprechenden Eingriffssituationen vergleichbarer medizinischer Eingriffe;
Ermitteln von Abweichungen der aktuellen Geräteeinstellungen von Geräteeinstellungen bei entsprechenden Eingriffssituationen vergleichbarer medizinischer Eingriffe; und
Ermitteln nötiger Anpassungen der aktuellen Geräteeinstellungen, um die Abweichungen zu kompensieren.

2. Verfahren nach Anspruch 1, wobei die Bestimmung der Eingriffssituation den Vergleich aktueller Daten mit historischen Daten umfasst.

3. Verfahren nach Anspruch 1 oder 2, wobei die Eingriffssituation auch auf den ausführenden Operateur abstellt.

4. Verfahren nach einem der vorstehenden Ansprüche, wobei die ermittelten nötigen Anpassungen wenigstens teilweise automatisch vorgenommen werden.

5. Verfahren nach Anspruch 4, wobei die automatische Vornahme der Anpassungen nach Freigabe erfolgt.

6. Verfahren nach einem der Ansprüche 1 bis 4, wobei die ermittelten nötigen Anpassungen auf einem Ausgabegerät angezeigt werden.

7. Verfahren nach einem der vorstehenden Ansprüche, wobei die Ermittlung von Abweichungen wenigstens teilweise auf einem durch künstliche Intelligenz gestützten Erkennungsalgorithmus erfolgt.

8. Verfahren nach Anspruch 7, wobei der Erkennungsalgorithmus mit historischen Daten trainiert wurde.

9. System umfassend wenigstens eine Datenquelle in einem integrierten Operationssaal, wenigstens ein konfigurierbares Gerät in dem integrierten Operationssaal, und wenigstens eine Datenverarbeitungseinheit, wobei das System konfiguriert ist, ein Verfahren gemäß einem der Ansprüche 1 bis 8 auszuführen.

10. Computerprogramm, umfassend Befehle, die bei der Ausführung des Programms durch einen Computer diesen veranlassen, die Verfahren gemäß einem der Ansprüche 1 bis 8 auszuführen.

11. Computerlesbares Speichermedium, umfassend Befehle, die bei der Ausführung durch einen Computer diesen veranlassen, die Verfahren gemäß einem der Ansprüche 1 bis 8 auszuführen.
